# EUROPEAN PATENT SPECIFICATION

(11) **EP 4 166 165 B1**
(45) Date of publication and mention of the grant of the patent: **30.07.2025**
(21) Application number: 22200249.5
(22) Date of filing: 07.10.2022
(51) Int. Cl.: B25J 5/00, B25J 11/00, B25J 19/02, G16H 30/40, G16H 40/63, G16H 40/67, A61L 2/24, A61L 2/10, B25J 13/08, G16H 40/20, G16H 40/40, A61L 9/20

(54) **METHODS OF SENSOR EXPOSURE VALIDATION**
VERFAHREN ZUR VALIDIERUNG DER SENSORBELICHTUNG
PROCÉDÉS DE VALIDATION D'EXPOSITION DE CAPTEUR

(30) Priority: 12.10.2021 US 202163254891 P; 04.10.2022 US 202217959411
(43) Date of publication of application: 19.04.2023
(73) Proprietor: Blue Ocean Robotics Aps, 5260 Odense (DK)
(72) Inventor: VISTISEN, Rasmus, Odense (DK); ØSTERGAARD, John Erland, Odense (DK); NIELSEN, Per Juul, Odense (DK); VITZRABIN, Efraim, Odense (DK)
(74) Representative: Cross, James Peter Archibald

(56) References cited:
- US-A1- 2014 212 332
- US-A1- 2020 230 273
- US-A1- 2021 299 868
- US-A9- 2019 117 813
- US-B2- 10 603 394

## Description

### BACKGROUND

Mobile devices, such as mobile robots, can be operated to disinfect areas of a room, such as a floor, walls, furniture, and the like that have a surface that is contaminated with pathogens. Typically, it is difficult to determine whether such mobile devices have disinfected all contaminated surfaces, or whether the disinfection has been effective. US 2021/299868 A1 discloses a method of plotting ultraviolet radiation for disinfection. US 2014/212332 A1 discloses an apparatus, system, and method for evaluating and adjusting the effectiveness of ultraviolet light disinfection of areas.

### BRIEF SUMMARY

According to an implementation of the disclosed subject matter, a method as set out in claim 1 is provided. Other aspects of the invention may be found in at least the dependent claims.

Additional features, advantages, and implementations of the disclosed subject matter may be set forth or apparent from consideration of the following detailed description, drawings, and claims. Moreover, it is to be understood that both the foregoing summary and the following detailed description are illustrative and are intended to provide further explanation without limiting the scope of the claims.

### BRIEF DESCRIPTION OF THE DRAWINGS

The accompanying drawings, which are included to provide a further understanding of the disclosed subject matter, are incorporated in and constitute a part of this specification. The drawings also illustrate implementations of the disclosed subject matter and together with the detailed description serve to explain the principles of implementations of the disclosed subject matter. No attempt is made to show structural details in more detail than may be necessary for a fundamental understanding of the disclosed subject matter and various ways in which it may be practiced.
FIG. 1 shows an example method of sensor exposure validation according to an implementation of the disclosed subject matter.
FIG. 2 shows a more detailed example of the method of FIG. 1 according to an implementation of the disclosed subject matter.
FIGS. 3-5 show a plurality of external views of the mobile robot having sensors to detect surfaces and objects in an area, and an ultraviolet (UV) light source to disinfect the air, objects, and/or surfaces in the area, and to expose one or more exposure sensors with UV light according to implementations of the disclosed subject matter.
FIG. 6 shows an example of the mobile robot detecting surfaces and/or objects of an area to disinfect them with the UV light according to an implementation of the disclosed subject matter.
FIG. 7 shows an example plotting of a representation of the emission of the UV light in the area according to an implementation of the disclosed subject matter.
FIG. 8 shows an example disinfection report (i.e., validation report) according to an implementation of the disclosed subject matter.
FIG. 9 shows an example configuration of the mobile robot of FIGS. 3-5 according to an implementation of the disclosed subject matter.
FIG. 10 shows a network configuration according to implementations of the disclosed subject matter.

### DETAILED DESCRIPTION

Implementations of the disclosed subject matter provide a mobile robot to perform disinfection of an area using ultraviolet (UV) light that is emitted by a light source disposed on the mobile robot, and to generate a disinfection exposure map of the portions of the area that have been disinfected. The disinfection by the mobile robot of the area and/or the disinfection exposure map may be validated by using one or more exposure sensors (e.g., UV light dosimeter) disposed in the area, which may change color or output a dosage level when exposed to the UV light emitted by the mobile robot. That is, information from the one or more exposure sensors may be used to validate the disinfection and/or the disinfection exposure map, and may be used to generate a report regarding the disinfection of the area by the mobile robot. The validation may demonstrate that the area has been successfully disinfected, and is safe to use. For example, a mobile robot may be deployed in a hospital, medical office, dental office, pharmaceutical and/or medical device manufacturing facility, and/or other settings such as an office, a warehouse, a school, a store, or the like to disinfect an area within these facilities, and may generate a report that includes an exposure map of the area, and validation of the exposure based on the output of one or more exposure sensors that are disposed in the area.

In some implementations, one or more exposure sensors (e.g., UV light dosimeters) may be placed in an area. The exposure sensors may be disposed at disinfection positions of interest in the area. For example, the positions of interest may be selected based on likelihood of contamination, based on activity within the area which may result in contamination. The mobile robot may output UV light from a light source to disinfect the area. In some implementations, an operator may capture images of the exposure sensors with a digital camera or image capture device, where the images may show a change in color of the exposure sensor (e.g., the exposure sensor changes from a first color to a second color based on the dosage of UV light) or a dosage value that is output by the exposure sensor (e.g., on a display of the exposure sensor). In some implementations, the operator may manually record a state (e.g., when the exposure sensor changes color) or a value output by the exposure sensor, and provide the recorded states and/or value to the mobile robot via the user interface of the mobile robot. In some implementations, the operator may provide the values from the exposure sensors to the mobile robot via a user interface and/or a communications interface (e.g., where the operator may transmit the values from a user device to the mobile robot via a wired and/or wireless communications link). In some implementations, the exposure sensor may provide a state value (whether the exposure sensor has changed colors or not) or a dosage value via a wireless communications link to the mobile robot. The mobile robot and/or the remote processor may generate a report that includes an exposure map of the area, and validation of the exposure based on the assigned UV exposure category for the exposure sensors.

In some implementations, an operator may assign positions on the exposure map to individual exposure sensors. The mobile robot may locate the exposure sensors that are dispersed in the area based on the assigned positions on the map, and may capture images of the exposure sensors (e.g., a change in color of the exposure sensor, or a value output by the exposure sensor that indicates dosage) before exposure of at least a portion of the area to UV light that is output by a light source disposed on the robot. In some implementations, the exposure sensors may transmit their positional location to the mobile robot via a wireless communications link. The mobile robot may assign the position of the exposure sensors on the map based on the transmitted positional location information. The mobile robot may perform a disinfection of the area by emitting UV light, and may capture images of the exposure sensors and/or may receive state values and/or dosage values from the exposure sensors after performing disinfection by using the assigned positions of the exposure sensors. The mobile robot may assign an actual UV exposure category (e.g., dosage amount) based on the images of the exposure sensors captured after disinfection (e.g., based on the change on color of the exposure sensor or a value outputted by the exposure sensor after being radiated with UV light), and/or may assign the actual UV exposure category based on the state value and/or dosage value received from the exposure sensors via the wireless communications link. The mobile robot and/or a remote processor may generate a report that includes an exposure map of the area, and validation of the exposure based on the images of the exposure sensors and/or the values from the exposure sensors (e.g., the state values and/or dosage values) to validate the exposure and/or the exposure map.

In some implementations, the mobile robot may identify the exposure sensors disposed within the area using sensors disposed on the mobile robot (e.g., at least one image sensor). The mobile robot may capture images of the exposure sensors (e.g., a change in color of the exposure sensor, or a value output by the exposure sensor that indicates a state value and/or dosage value) before exposure of at least a portion of the area to UV light that is output by a light source disposed on the robot. The mobile robot may perform a disinfection of the area by emitting UV light, and may capture images of the exposure sensors after performing disinfection. The mobile robot may assign an actual UV exposure category (e.g., dosage amount) based on the images of the exposure sensors captured after disinfection (e.g., by an image sensor of the mobile robot). The mobile robot and/or a remote processor may generate a report that includes an exposure map of the area, and validation of the exposure based on the images of the exposure sensors or the values from the exposure sensors to validate the exposure and/or the exposure map.

In some implementations, the mobile robot may perform a disinfection of the area by emitting UV light, and may capture images of the exposure sensors after performing a disinfection operation. If the captured images of the exposure sensors do not show a UV exposure category of at least a predetermined amount, the mobile robot may perform additional disinfection of at least a portion of the area by emitting UV light from the light source. The mobile robot may capture images of the exposure sensors after performing the additional disinfection to determine whether the exposure sensors show the predetermined amount of a UV exposure category. The mobile robot and/or a remote processor may generate a report that includes an exposure map of the area, and validation of the exposure based on the images of the exposure sensors or the values (e.g., a state value and/or dosage value) from the exposure sensors to validate the exposure and/or the exposure map.

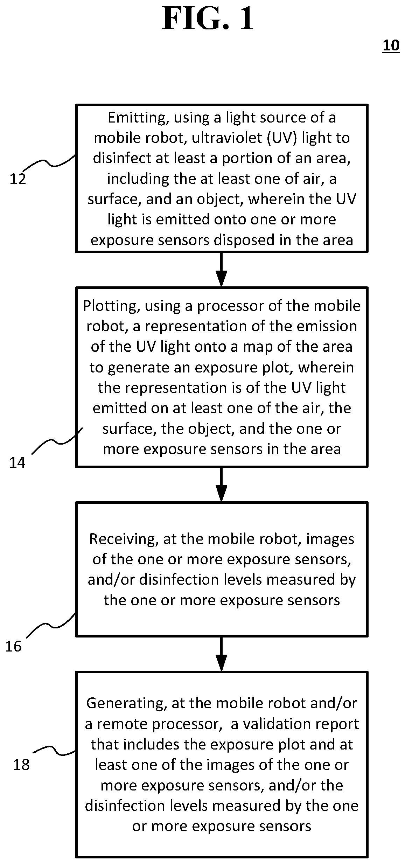

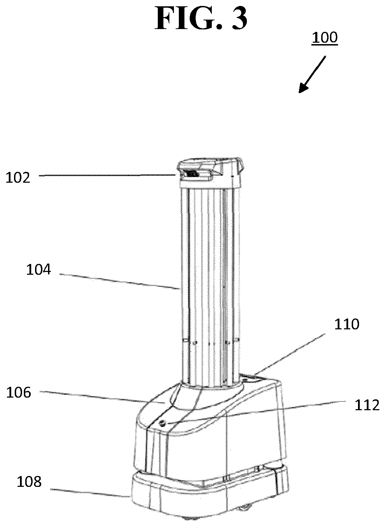

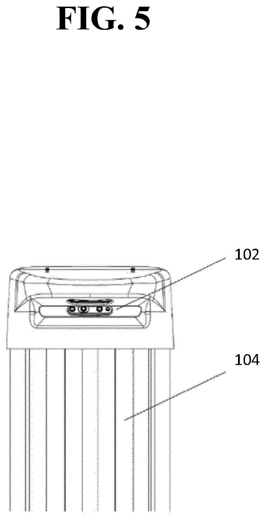

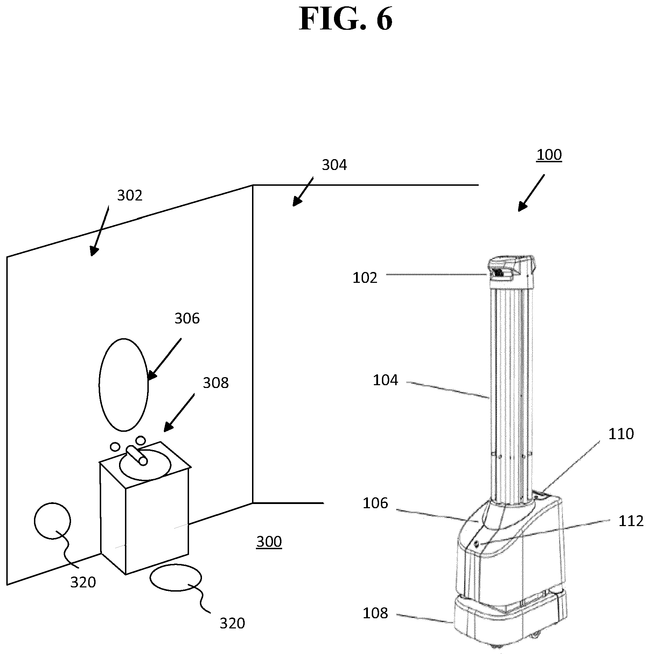

FIG. 1 shows an example method 10 of sensor exposure validation according to an implementation of the disclosed subject matter. At operation 10, a light source (e.g., light source 104 shown in FIGS. 3-6) of a mobile robot (e.g., mobile robot 100 shown in FIGS. 3-6, 9, and 10), may emit ultraviolet (UV) light to disinfect at least a portion of an area (e.g., surface 300, 302, and/or 304, and/or object 306, 308 shown in FIG. 6), including air, a surface, and/or an object. The UV light may be emitted onto one or more exposure sensors (e.g., exposure sensor 320 shown in FIG. 6) disposed in the area.

For example, the exposure sensor may be a UV light dosimeter which may change color based on the dose of UV radiation that is delivered to the exposure sensor. That is, the exposure sensor may change from a first color to a second color when the dosage of UV light received by the exposure sensor is greater than or equal to a predetermined amount. In another example, the exposure sensor may display a dosage value of UV radiation that the sensor has received. In yet another example, the exposure sensor may transmit a dosage value and/or a state value (e.g., where the state value represents a color of the exposure sensor) via a wireless communications link to the mobile robot. In some implementations, an operator may place the one or more exposure sensors in the area at relevant disinfection positions (e.g., surfaces and/or areas that may be most likely to be contaminated, such as shown by exposure sensors 320 in FIG. 6).

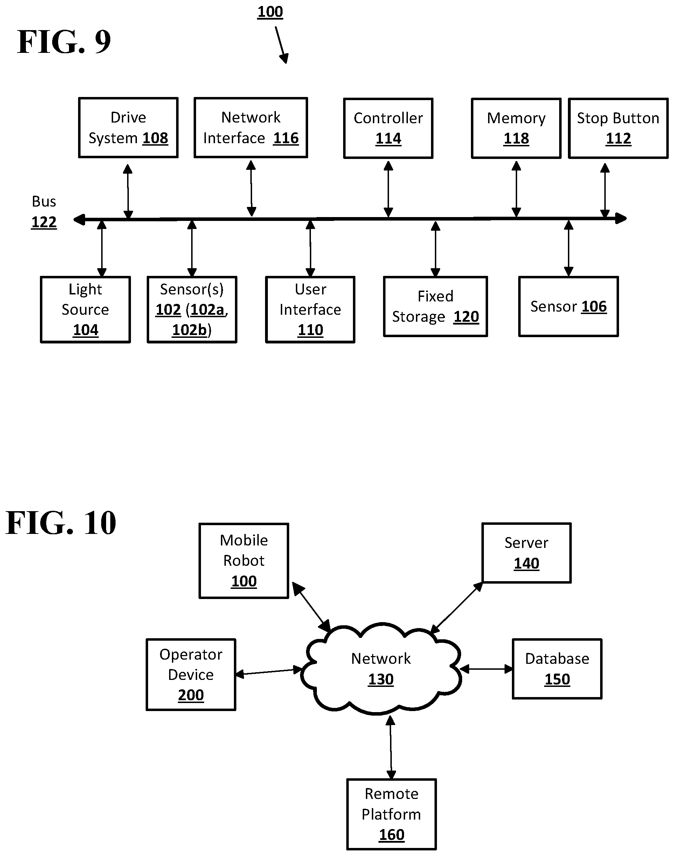

In some implementations, positions of the one or more exposure sensors may be assigned on an electronic map of the area, which may be stored in memory 118 and/or fixed storage 120 shown in FIG. 9, and/or stored at server, 140, database 150, and/or remote platform 160 shown in FIG. 10. The mobile robot may determine (e.g., using the controller 114 and/or sensors 102, 102a, 102b) a location of the one or more exposure sensors in the area based on images and/or data from the sensors 102, 102a, 102b and/or based on the map that includes the assigned positions. In some implementations, the mobile robot may determine the positions of the one or more exposure sensors 320 using sensors 102, 102a, and/or 102b shown in FIGS. 3-6 and 9. The determined positions of the exposure sensors may be added to the map, and stored in memory (e.g., memory 118 and/or fixed storage 120 shown in FIG. 9, and/or stored at server, 140, database 150, and/or remote platform 160 shown in FIG. 10). In some implementations, the exposure sensors (e.g., exposure sensors 320) may transmit their positional location to the mobile robot via a wireless communications link (e.g., via network 130 shown in FIG. 10). The mobile robot may assign the position of the exposure sensors on the map based on the transmitted positional location information, and the position of the exposure sensors may be stored in memory (e.g., memory 118 and/or fixed storage 120 shown in FIG. 9, and/or stored at server, 140, database 150, and/or remote platform 160 shown in FIG. 10).

At operation 14, the processor of the mobile robot (e.g., controller 114 shown in FIG. 9) may plot a representation of the emission of the UV light onto a map of the area to generate an exposure plot. The representation may be of the UV light emitted on at least one of the air, the surface, the object, and the one or more exposure sensors in the area. The plot may be generated as the mobile robot emits UV light in the area, either while the mobile robot is stationary or while the mobile robot moves within the area.

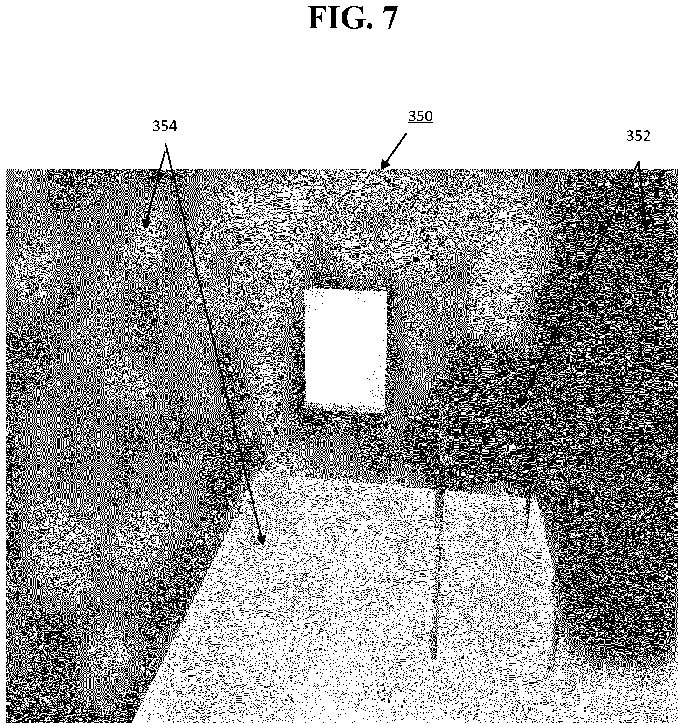

An example of the exposure plot (e.g., exposure plot 350) is shown in FIG. 7. In the exposure plot 350, areas 352 may be areas that have been disinfected by UV light from the light source of the mobile robot. For example, the darker the areas 352 may be in the exposure plot 350, the more UV light that the surface and/or object may have received. Areas 354 may not have received as much UV light exposure as areas 352. For example, as shown in the exposure plot 350, the areas 354 are less dark than those of areas 352, which may indicate that they have received less UV light.

At operation 16, the mobile robot may receive at least one of image of the one or more exposure sensors (e.g., exposure sensors 320 shown in FIG. 6), and/or disinfection levels measured by the one or more exposure sensors (e.g., a state value and/or a dosage value). The images may be received by, for example, sensors 102, 102a, 102b. The received images may show when the exposure sensors (e.g., UV light dosimeters) have changed color based on the UV light emitted by the mobile robot, and/or display a changed dosage value of UV radiation that the exposure sensor has received. The state value may be representative of a color change of the exposure sensor, and the dosage value may be the amount of UV dosage that the exposure sensor received.

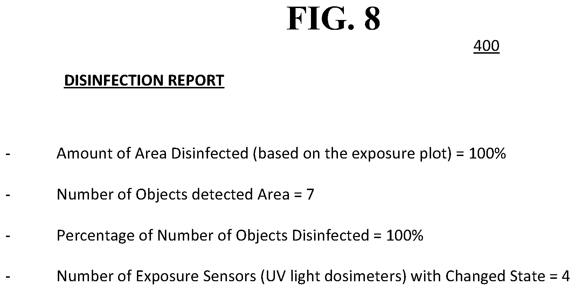

At operation 18, the mobile robot and/or a remote processor (e.g., server 140 and/or remote platform 160 shown in FIG. 10, or the like) may generate a validation report that includes the exposure plot and at least one of the images of the one or more exposure sensors, and/or the disinfection levels measured by the one or more exposure sensors, and/or the state values and/or dosage values of the exposure sensor. That is, the generated report may include the exposure plot, along with images and/or data (e.g., state values and/or dosage values) from the one or more exposure sensors to verify the disinfection of the area. The generated validation report may be transmitted via a communications network (e.g., communications network 130 shown in FIG. 10) from the mobile robot to server 140, database 150, remote platform 160 and/or operator device 200. In another example, the generated validation report may be transmitted via a communications network from the mobile robot and/or server 140 and/or remote platform 160 to operator device 200. An example of the report is shown in FIG. 8 and described below.

In some implementations, the mobile robot (e.g., controller 114 shown in FIG. 9) may assign an actual exposure amount of a portion of the area based on an output from the one or more exposure sensors to the UV light. The actual exposure amount may be used by the mobile robot and/or the remote processor to generate the validation report. The actual exposure value may be received by the network interface 116 and/or the sensor 102, 102a, 102b. When the validation report is generated at least in part by the server 140 and/or the remote platform 160, it may be transmitted via the network 130 to the database 150 to be stored.

In some implementations, the mobile robot or a remote processor (e.g., operator device 200, server 140, and/or remote platform 160 shown in FIG. 10) may determine a future disinfection plan for the mobile robot based on a comparison between the exposure plot and at least one of received images and/or disinfection levels of sensor 102, 102a, 102b. The future disinfection plan may be used by the mobile robot to disinfect at least a portion of the area.

In some implementations, the mobile robot may receive images of the one or more exposure sensors (e.g., exposure sensors 320), and/or disinfection levels (e.g., state values and/or dosage values) measured by the one or more exposure sensors before and after the emission of UV light in the area. The images and/or disinfection levels (e.g., state values and/or dosage values) may be provided by a device of the operator (e.g., operator device 200 shown in FIG. 10) that is communicatively coupled to the mobile robot. In some implementations, the mobile robot may capture images of the one or more exposure sensors and/or disinfection levels output by the exposure sensors using sensors 102, 102a, and/or 102b shown in FIGS. 3-6. In some implementations, the communication interface (e.g., network interface 116 shown in FIG. 9) of the mobile robot may receive dosage levels (e.g., state values and/or dosage values) that are transmitted by the one or more exposure sensors (e.g., exposure sensors 320). The mobile robot may determine whether to continue to emit UV light (e.g., from the light source 104) based on the images of the one or more exposure sensors (e.g., that include the output of the exposure sensors, such as a change in color) and/or disinfection levels (e.g., state values and/or dosage values) measured by the one or more exposure sensors before and after the emission of UV light in the area and transmitted to the mobile robot via a communications link. That is, the mobile robot may determine whether the disinfection levels are greater than or equal to a predetermined level. The mobile robot may continue to perform disinfection of the area by emitting UV light from the light source when the disinfection levels are less than the predetermined level and may stop the disinfection when the levels are greater than or equal to the predetermined level.

In some implementations, the mobile robot or a remote processor (e.g., operator device 200, server 140, and/or remote platform 160 shown in FIG. 10) may determine a future disinfection plan for the mobile robot based on a comparison between the exposure plot and the at least one of the images of the one or more exposure sensors (e.g., exposure sensors 320) and/or disinfection levels measured by the one or more exposure sensors (e.g., using sensors 102, 102a, and/or 102b). The future disinfection plan may be used by the mobile robot to disinfect at least a portion of the area.

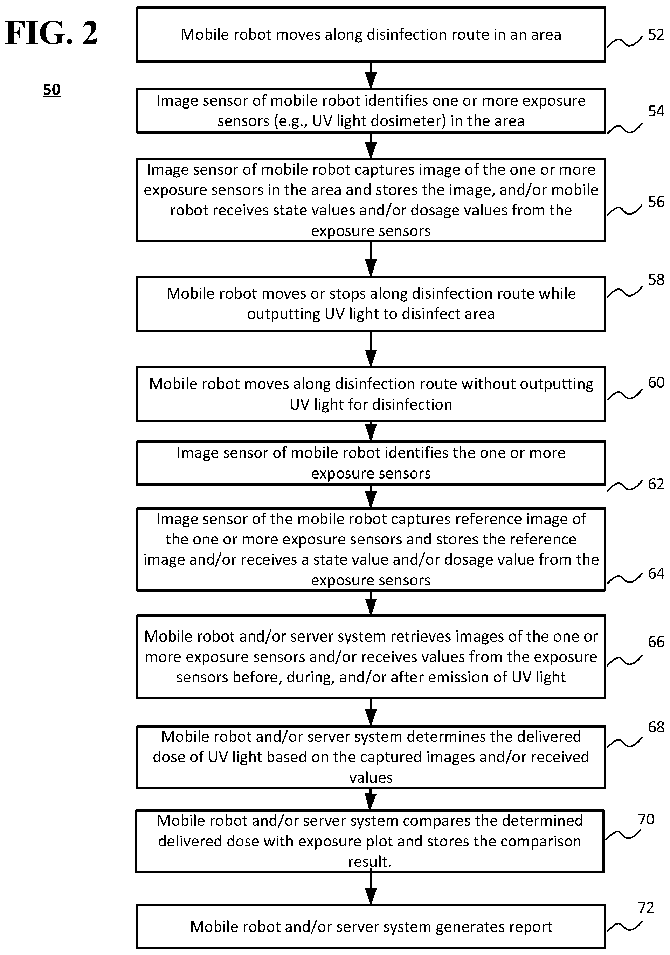

FIG. 2 shows a more detailed example method 50 of the method 10 shown in FIG. 1 according to an implementation of the disclosed subject matter. At operation 52, the mobile robot (e.g., mobile robot 100) may move or stop along a disinfection route in an area. The mobile robot may move or stop autonomously along the disinfection route, and/or may receive control signals via a communications interface (e.g., network interface 116 shown in FIG. 9) to move or stop along the disinfection route. The disinfection route may be included in a map that is stored in the memory of the mobile robot, and/or is accessible from the server 140, database 150, and/or remote platform 160 via a communications interface of the mobile robot. The controller 114 may control the drive system 108 to move the mobile robot 100 according to the disinfection route.

At operation 54, an image sensor (e.g., sensor 102, 102a, 102b shown in FIGS. 3-5) of the mobile robot may identify one or more exposure sensors (e.g., UV light dosimeter, such as exposure sensor 320) in the area. At operation 56, the image sensor of the mobile robot may capture images of the one or more exposure sensors in the area, and/or may receive state values and/or dosage values from the one or more exposure sensors via a communications link. The captured images, state values, and/or dosage values may be stored, for example, in memory 118 and/or fixed storage 120 shown in FIG. 9, and/or at database 150 shown in FIG. 10.

At operation 58, the mobile robot may move or stop along the disinfection route and may output UV light to disinfect the area. The mobile robot may move or stop autonomously along the disinfection route, and/or may receive control signals via a communications interface (e.g., network interface 116 shown in FIG. 9) to move or stop along the disinfection route. The controller 114 may control the drive system 108 to move or stop the mobile robot along with disinfection route and may control the output of UV light from the light source 104. At operation 60, the mobile robot may continue to move along the disinfection route but may not output UV light. The controller 114 may control the light source 104 to stop emitting light and may control the drive system 108 to continue to move the mobile robot. That is, the mobile robot may move without emitting UV light to travel to another portion of the area (e.g., that includes exposure sensors 320) to perform disinfection.

At operation 62, the image sensor (e.g., sensor 102, 102a, 102b shown in FIGS. 3-5) of the mobile robot may identify the one or more exposure sensors (e.g., the UV light dosimeters). At operation 64, the image sensor of the mobile robot may capture a reference image of the one or more exposure sensors (e.g., UV light dosimeters) receive a state value (e.g., the color of the exposure sensor, and whether it has changed color) from the exposure sensor, and/or receive a dosage value from the exposure sensor. The reference image, received state value, and/or received dosage value may be stored in memory 118 and/or fixed storage 120 shown in FIG. 9, and/or at database 150 shown in FIG. 10.

At operation 66, the mobile robot and/or server system (e.g., server 140 and/or the remote platform 160) may receive images of the one or more exposure sensors (e.g., UV light dosimeters), receive state values of the one or more exposure sensors, and/or receive dosage values of the one or more exposure sensors before, during, and/or after the emission of the UV light. The images may be received, and/or the state values and/or dosage values may be received, from the operator device 200 via a communications link. Alternatively, the mobile robot may capture images and/or receive the state values and/or dosage values before, during, and/or after the emission of UV light, and may retrieve the captured images, the state values, and/or dosage values from memory (e.g., memory 118 and/or fixed storage 120 shown in FIG. 9, and/or the server 140, database 150, and/or remote platform 160 shown in FIG. 10). For example, by capturing images and/or receiving the state values and/or dosage values during the emission of UV light, the controller may control the stopping of the emission of UV light when a particular surface and/or area has been disinfected.

At operation 68, the mobile robot and/or server system (e.g., server 140 and/or remote platform 160 shown in FIG. 10) may determine the delivered dose of UV light based on the captured images of the one or more exposure sensors. In some implementations, the delivered dose may be determined from the state value and/or dosage values transmitted to the mobile robot and/or server system from the one or more exposure sensors. At operation 70, the mobile robot and/or server system may compare the determined delivered dose with the exposure plot. The result of the comparison may be stored in memory 118 and/or fixed storage 120 shown in FIG. 9, and/or at database 150 shown in FIG. 10. At operation 72, the mobile robot and/or server system may generate a report, which may detail the areas that have been disinfected, and verification of the disinfection (e.g., pictures of the exposure sensors, and/or state values and/or dosage values of the exposure sensors). An example report is shown in FIG. 8 and described below. In some implementations, the mobile robot and/or a remote processor (e.g., operator device 200, server 140, and/or remote platform 160 shown in FIG. 10) a future disinfection plan for the mobile robot based on the stored comparison. The future disinfection plan may be used by the mobile robot to disinfect at least a portion of the area.

The mobile robot may reduce human error in disinfecting an area, room, building, or the like by tracking the location and/or intensity (e.g., optical power of UV light) of light radiated, and determine which areas may need to be radiated and/or disinfected based on the position of the exposure sensors. The representation of the emission of the UV may be based on a light emission model used by the processor, where the UV light emitted is based on a square of a distance from the light source. The exposure plot (e.g., exposure plot 350 shown in FIG. 7) may be generated by the processor during the emission of the UV light or after disinfection of at least one of the air, the surface, and/or the object of the area. In some implementations, the plotting of the emitted UV light onto the mapped area may be performed after the area is mapped and disinfected.

In some implementations, the method 10 or the method 50 may include transmitting a disinfection report using a communications interface of the mobile robot (e.g., network interface 116 of the mobile robot 100 shown in FIG. 9 may transmit via the network 130 shown in FIG. 10). An example disinfection report 400 is shown in FIG. 8. The disinfection report may include, for example, an amount of the area that has been disinfected based at least on the exposure plot, a number objects detected in the area, a percentage of the number of objects disinfected based on the detected number of objects and the exposure plot, a number of exposure sensors (UV light dosimeters) that have a changed stated based on exposure to the UV light, a path of the mobile robot moving in the area, and/or one or more deviations from a planned path of the mobile robot moving in the area, or the like.

As shown in the disinfection report 400 of FIG. 8, an amount of area disinfected by the mobile robot may be 100%, based on the exposure plot generated by the processor. In the example shown in FIG. 8, there may be seven (7) objects detected in the area by the one or more sensors of the mobile robot (e.g., sensors 102 and/or 106). The percentage of the number of objects disinfected may be 100%, which may be based on the number of objects detected, and the generated exposure plot (e.g., exposure plot 350 shown in FIG. 7). As shown in the disinfection report 400 of FIG. 8, there may be four (4) exposure sensors (UV light dosimeters) that have a changed state. That is, as described above, the exposure sensor may change from a first state to a second state when it receives UV light from the light source of the mobile robot. In some implementations, the disinfection report (e.g., disinfection report 400) may be stored in at least one of a storage device (e.g., memory 118 and/or fixed storage 120 shown in FIG. 9), a cloud storage device (e.g., at server 140 and/or remote platform 160 shown in FIG. 10), and/or a database system (e.g., database 150 shown in FIG. 10).

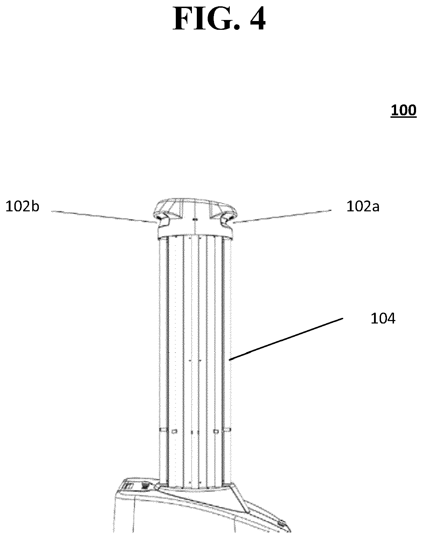

FIGS. 3-5 show a plurality of external views of a mobile robot 100 that includes sensors to detect surfaces and objects in an area, and an ultraviolet (UV) light source to disinfect the air, objects, and/or surfaces in the area according to implementations of the disclosed subject matter. The mobile robot 100 may include at least a first sensor 102 (shown as sensor 102a and 102b in FIG. 4), a light source 104 to output ultraviolet light, at least a second sensor 106, a drive system 108, a user interface 110, and/or a stop button 112. A controller (e.g., controller 114 shown in FIG. 9 and described below) may be communicatively coupled to the at least one first sensor 102, the light source 104, the at least one second sensor 106, the drive system 108, the user interface 110 and the stop button 112, may control the operations of the mobile robot 100.

The at least one first sensor 102 (including sensors 102a, 102b shown in FIG. 4) may determine at least one of an orientation of the mobile robot 100 (e.g., a direction that a front side and/or a first side of a robot is facing), a location of the mobile robot 100 (e.g., a location of the mobile robot 100 in an area), and/or when the light source 104 is within a predetermined distance of a surface and/or object in the area (e.g., surface 300, 302, and/or 304, and/or object 306, 308 shown in FIG. 6). In some implementations, the first sensor 102 may detect air, a surface, one or more exposure sensors (UV light dosimeters), and/or objects that may be mapped by the mobile robot 100 and/or disinfected with UV light from the light source 104.

In some implementations, the at least one first sensor 102 may have a field of view of 70 degrees diagonally. The at least one sensor 102 may have a detection distance of 0.2 - 4 meters. As shown in FIGS. 3-5, the at least one first sensor 102 may be disposed over the light source 104.

The at least one first sensor 102 may include a first side sensor disposed on a first side of the mobile robot 100 and a second side sensor that may be disposed on a second side of the device. For example, as shown in FIG. 4, sensor 102a may be disposed on a first side (e.g., a front side) of the mobile robot 100, and sensor 102b may be disposed on a second side (e.g., a back side) of the mobile robot 100. Although sensors on two sides of the robot are shown in FIG. 4, there may be a plurality of sensors disposed on different sides of the mobile robot 102 to at least detect surfaces and/or objects. In some implementations, sensor 102a and/or sensor 102b may be disposed over the light source 104.

The light source 104 may be one or more bulbs, one or more lamps, and/or an array of light emitting diodes (LEDs) or organic light emitting diodes (OLEDs) to emit UV light (e.g., light having a wavelength of 10 nm - 400 nm). The intensity (i.e., optical power output) may be controlled by the controller 114, which may also turn on or off a portion or all of the devices (e.g., bulbs, lamps, LEDs, OLEDs) of the light source 104. The light source may be controlled to emit UV light when the mobile robot is within an area, as the mobile robot moves within the area, before the mapping of the area, during the mapping of the area, and/or after the mapping of the area.

The at least one second sensor 106 may be communicatively coupled to the controller 114 shown in FIG. 9, and may be used to detect air, surfaces, and/or objects that may be mapped and/or disinfected with UV light from the light source 104. In some implementations, the at least one second sensor 106 may determine at least one of an orientation of the mobile robot 100 (e.g., a direction that a front side and/or a first side of a robot is facing), a location of the mobile robot 100 (e.g., a location of the mobile robot 100 in an area), and/or when the light source 104 is within a predetermined distance of a surface and/or object in the area (e.g., surface 300, 302, and/or 304, and/or object 306, 308 shown in FIG. 6).

In some implementations, the sensor 102, 106 may be a time-of-flight sensor, an ultrasonic sensor, a two-dimensional (2D) Light Detection and Ranging (LiDAR) sensor, a three-dimensional (3D) LiDAR sensor, and/or a radar (radio detection and ranging) sensor, a stereo vision sensor, 3D three camera, a structured light camera, or the like. The sensor 106 may have a field of view of 20-27 degrees. In some implementations, the sensor 106 may have a detection distance of 0.05 - 4 meters.

The mobile robot 100 may include a motor to drive the drive system 108 to move the mobile robot in an area, such as a room, a building, or the like. The drive system 108 may include wheels, which may be adjustable so that the drive system 108 may control the direction of the mobile robot 100.

In some implementations, the mobile robot 100 may include a base with the drive system 108, and the sensor 102, 106 may be disposed on the base.

The controller 114 may control and/or operate the mobile robot 100 in an operation mode which may be a manual mode, an autonomous mode, and/or a tele-operation mode. In the manual mode, the controller 114 may receive one or more control signals from the user interface 110 and/or the stop button 112. For example, a user may control the movement, direction, and/or stop the motion of the mobile robot 100 by making one or more selections on the user interface 110. The stop button 112 may be an emergency stop (ESTOP) button which may stop all operations and/or movement of the mobile robot 100 when selected. In some implementations, the controller 114 may receive at least one control signal via a network interface 116 (shown in FIG. 9) when operating in the tele-operation mode. For example, the network interface may receive control signals via network 130 from server 140, database 150, and/or remote platform 160, as described below in connection with FIG. 10.

In some implementations, when the mobile robot 100 is moving in a direction, the sensor 102, 106 may detect a geometry of one or more surfaces (e.g., surfaces 300, 302, 304 shown in FIG. 6) and/or objects (e.g., objects 306, 308 and/or sensor 320 shown in FIG. 6). The output of the at least one first sensor 102 may be, for example, a point cloud of the one or more objects in the path of the mobile robot 100. When the sensor 102 and/or sensor 106 is a stereo vision sensor, images from two sensors (i.e., where the two sensors may be part of the stereo vision sensor of the sensor 102 and/or sensor 106) within a known distance from one another distance may be captured at a predetermined point in time, and/or at predetermined time intervals with a global shutter. The global shutter may be configured so that the two sensors of the stereo vision sensor may capture images about simultaneously. One or more features (e.g., surfaces 300, 302, 304, and/or objects 306, 308 and/or sensor 320 shown in FIG. 6) may be determined from the captured images and be compared to one another to determine portions that are matching. As the focal length of the two sensors of the stereo vision sensor and the distance between the two sensors (e.g., about 6 cm) may be stored in memory 118 and/or fixed storage 120 (shown in FIG. 12), the controller 114 and/or the at least one first sensor 102 may use the captured images and the stored values to determine the distance from the sensor 102, 106 to the surfaces and/or objects, and may be used by the processor for mapping (as described above in connection with FIG. 1). In some implementations, the sensor 102, 106 may include at least one laser, LED, and/or OLED, to radiate one or more points on surfaces of objects, when the objects may be without identifying features (e.g., blank walls).

When detecting the surface and/or object, the sensor 102, 106 may be a time-of-flight (TOF) sensor. At least one photon of light may be output by the sensor 102, 106, and may be transmitted through the air. When the at least one photon of light radiates surface and/or an object, a portion of the light may be reflected by the surface and/or the object may return to a receiver portion of the sensor 102, 106. The sensor 106 may calculate the time between sending the at least one photon of light and receiving the reflection and multiply this value by the speed of light in air, to determine the distance between the sensor 102, 106 and surface and/or object. This may be used to generate the map of the area that the mobile robot is operating within.

An exposure calculation model may determine an intensity and/or duration of UV light to emit from the light source 104 to disinfect air, surfaces, and/or objects within a room and/or area.

FIG. 9 shows example components of the mobile robot 100 suitable for providing the implementations of the disclosed subject matter. The mobile robot 100 may include a bus 122 which interconnects major components of the mobile robot 100, such as the drive system 108, a network interface 116 operable to communicate with one or more remote devices via a suitable network connection, the controller 114, a memory 118 such as Random Access Memory (RAM), Read Only Memory (ROM), flash RAM, or the like, the stop button 112, the light source 104, the at least one first sensor 102, a user interface 110 that may include one or more controllers and associated user input devices such as a keyboard, touch screen, and the like, a fixed storage 120 such as a hard drive, flash storage, and the like, and the at least one second sensor 106.

The bus 122 allows data communication between the controller 114 and one or more memory components, which may include RAM, ROM, and other memory, as previously noted. Typically, RAM is the main memory into which an operating system and application programs are loaded. A ROM or flash memory component can contain, among other code, the Basic Input-Output system (BIOS) which controls basic hardware operation such as the interaction with peripheral components. Applications resident with the mobile robot 100 are generally stored on and accessed via a computer readable medium (e.g., fixed storage 120), such as a solid-state drive, hard disk drive, an optical drive, solid state drive, or other storage medium.

The network interface 116 may provide a direct connection to a remote server (e.g., server 140, database 150, and/or remote platform 160 shown in FIG. 10) via a wired or wireless connection (e.g., network 130 shown in FIG. 10). The network interface 116 may provide such connection using any suitable technique and protocol as will be readily understood by one of skill in the art, including digital cellular telephone, WiFi, Bluetooth(R), near-field, and the like. For example, the network interface 116 may allow the mobile robot 100 to communicate with other computers via one or more local, wide-area, or other communication networks, as described in further detail below. The mobile robot may transmit data via the network interface to the remote server that may include a path of operation, the surfaces and/or areas radiated with UV light, and the like.

Many other devices or components (not shown) may be connected in a similar manner. Conversely, all of the components shown in FIG. 9 need not be present to practice the present disclosure. The components can be interconnected in different ways from that shown. Code to implement the present disclosure can be stored in computer-readable storage media such as one or more of the memory 118, fixed storage 120, or on a remote storage location.

FIG. 10 shows an example network arrangement according to an implementation of the disclosed subject matter. Mobile robot 100 described above, may connect to other devices via network 130. The network 130 may be a local network, wide-area network, the Internet, or any other suitable communication network or networks, and may be implemented on any suitable platform including wired and/or wireless networks. The mobile robot 100 and/or mobile robot 200 may communicate with one another, and/or may communicate with one or more remote devices, such as server 140, database 150, remote platform 160, and/or operator device 200. The remote devices may be directly accessible by the mobile robot 100 or one or more other devices may provide intermediary access such as where a server 140 provides access to resources stored in a database 150. The mobile robot 100 may access remote platform 160 or services provided by remote platform 160 such as cloud computing arrangements and services. The remote platform 160 may include one or more servers 140 and/or databases 150. The operator device 200 may be a computer, smartphone, wearable computing device, or the like.

More generally, various implementations of the presently disclosed subject matter may include or be embodied in the form of computer-implemented processes and apparatuses for practicing those processes. Implementations also may be embodied in the form of a computer program product having computer program code containing instructions embodied in non-transitory and/or tangible media, such as solid state drives, DVDs, CD-ROMs, hard drives, USB (universal serial bus) drives, or any other machine readable storage medium, such that when the computer program code is loaded into and executed by a computer, the computer becomes an apparatus for practicing implementations of the disclosed subject matter. Implementations also may be embodied in the form of computer program code, for example, whether stored in a storage medium, loaded into and/or executed by a computer, or transmitted over some transmission medium, such as over electrical wiring or cabling, through fiber optics, or via electromagnetic radiation, such that when the computer program code is loaded into and executed by a computer, the computer becomes an apparatus for practicing implementations of the disclosed subject matter. When implemented on a general-purpose microprocessor, the computer program code segments configure the microprocessor to create specific logic circuits.

In some configurations, a set of computer-readable instructions stored on a computer-readable storage medium may be implemented by a general-purpose processor, which may transform the general-purpose processor or a device containing the general-purpose processor into a special-purpose device configured to implement or carry out the instructions. Implementations may include using hardware that has a processor, such as a general-purpose microprocessor and/or an Application Specific Integrated Circuit (ASIC) that embodies all or part of the techniques according to implementations of the disclosed subject matter in hardware and/or firmware. The processor may be coupled to memory, such as RAM, ROM, flash memory, a hard disk or any other device capable of storing electronic information. The memory may store instructions adapted to be executed by the processor to perform the techniques according to implementations of the disclosed subject matter.

The foregoing description, for purpose of explanation, has been described with reference to specific implementations. However, the illustrative discussions above are not intended to be exhaustive or to limit implementations of the disclosed subject matter to the precise forms disclosed.

## Claims

1. A method comprising:
emitting, using a light source of a mobile robot, ultraviolet, UV, light to disinfect at least a portion of an area, including the at least one of a surface and an object, wherein the UV light is emitted onto one or more exposure sensors disposed in the area;
plotting, using a processor of the mobile robot, a representation of the emission of the UV light onto a map of the area to generate an exposure plot, wherein the representation is of the UV light emitted on at least one of the surface, the object, and the one or more exposure sensors in the area, said representation of the emission of the UV light is based on a light emission model used by the processor;
receiving, at the mobile robot, at least one from the group consisting of: images of the one or more exposure sensors, and disinfection levels measured by the one or more exposure sensors, the one or more exposure sensors change in color or output a dosage value based on dosage of UV light received; and
generating, using at least at one selected from the group consisting of:
(i) the mobile robot, and
(ii) a remote processor a validation report **characterized in that** it includes at least the exposure plot, wherein the exposure plot is validated by comparing the exposure plot and at least one selected from the group consisting of: the images of the one or more exposure sensors that are captured by an image sensor of the mobile robot, and the disinfection levels measured by the one or more exposure sensors that are captured by the image sensor or received by a network interface of the mobile robot via a communications link with the one or more exposure sensors based on exposure to the UV light emitted by light source of a mobile robot.

2. The method of claim 1, further comprising,
controlling, at the processor of the mobile robot, a drive system of the mobile robot to move or stop the mobile robot by at least one selected from the group consisting of:
autonomously moving or stopping the mobile robot, and moving or stopping the mobile robot by control signals received via a communications interface along a disinfection route in the area.

3. The method of claim 2, wherein the emitting the UV light to disinfect at least a portion of the area comprises:
emitting UV light as the mobile robot moves along the disinfection route.

4. The method of claim 1, further comprising:
assigning, at the processor of the mobile robot, positions of the one or more exposure sensors on the map of the area.

5. The method of claim 1, wherein the disinfection levels of the one or more exposure sensors are received during at least one time selected from the group consisting of: before the emission of UV light in the area, during the emission of UV light in the area, and after the emission of UV light in the area.

6. The method of claim 1, further comprising:
assigning, at the mobile robot, an actual exposure amount of the one or more exposure sensors to the UV light based on at least one of the received images and the disinfection levels; and
determining, at the mobile robot or the remote processor, a future disinfection plan for the mobile robot based on a comparison between the exposure plot and at least one of the received images and the disinfection levels.

7. The method of claim 1, further comprising:
determining, at the mobile robot, whether to continue to emit UV light based on the at least one of the images of the one or more exposure sensors and disinfection levels measured by the one or more exposure sensors; and
determining, at the mobile robot or the remote processor, a future disinfection plan for the mobile robot based on a comparison between the exposure plot and the at least one of the images of the one or more exposure sensors and disinfection levels measured by the one or more exposure sensors.

8. The method of claim 1, further comprising:
determining a delivered dose of UV light based on at least one selected from the group consisting of: the received images of the one or more exposure sensors, and the received disinfection levels.

9. The method of claim 1, further comprising:
comparing, at the processor of the mobile robot or a server system communicatively coupled to the mobile robot, a determined delivered dose with the exposure plot;
storing a result of the comparison in a memory communicatively coupled to at least one selected from the group consisting of: the mobile robot, and the server system; and
determining, at the mobile robot or the remote processor, a future disinfection plan for the mobile robot based on the stored comparison.

## Patentansprüche

1. Verfahren, das Folgendes beinhaltet:
Emittieren, mittels einer Lichtquelle eines mobilen Roboters, von ultraviolettem (UV) Licht zum Desinfizieren mindestens eines Teils eines Bereichs, der eine Oberfläche und/oder ein Objekt umfasst, wobei das UV-Licht auf einen oder mehrere in dem Bereich angeordnete Belichtungssensoren emittiert wird;
Plotten, mittels eines Prozessors des mobilen Roboters, einer Darstellung der Emission des UV-Lichts auf eine Karte des Bereichs, um ein Belichtungsdiagramm zu erzeugen, wobei die Darstellung das auf mindestens eines von der Oberfläche, dem Objekt und den ein oder mehreren Belichtungssensoren in dem Bereich emittierte UV-Licht darstellt, wobei die genannte Darstellung der Emission des UV-Lichts auf einem von dem Prozessor verwendeten Lichtemissionsmodell basiert;
Empfangen, an dem mobilen Roboter, von mindestens einem Element aus der Gruppe bestehend aus: Bildern der ein oder mehreren Belichtungssensoren und von den ein oder mehreren Belichtungssensoren gemessenen Desinfektionsgraden, wobei die ein oder mehreren Belichtungssensoren ihre Farbe ändern oder einen Dosierungswert auf der Basis der empfangenen UV-Lichtdosis ausgeben; und
Erzeugen eines Validierungsberichts unter Verwendung von mindestens einem Element ausgewählt aus der Gruppe bestehend aus:
(i) dem mobilen Roboter und
(ii) einem Fernprozessor
**dadurch gekennzeichnet, dass** ein Validierungsbericht mindestens das Belichtungsdiagramm enthält, wobei das Belichtungsdiagramm durch Vergleichen des Belichtungsdiagramms und mindestens eines Elements ausgewählt aus der Gruppe bestehend aus: den von einem Bildsensor des mobilen Roboters aufgenommenen Bildern der ein oder mehreren Belichtungssensoren und den von den ein oder mehreren Belichtungssensoren gemessenen Desinfektionsgraden validiert wird, die von dem Bildsensor aufgenommen werden oder von einer Netzwerkschnittstelle des mobilen Roboters über eine Kommunikationsverbindung mit den ein oder mehreren Belichtungssensoren auf der Basis der Belichtung mit dem von einer Lichtquelle eines mobilen Roboters emittierten UV-Licht empfangen werden.

2. Verfahren nach Anspruch 1, das ferner Folgendes beinhaltet:
Steuern, an dem Prozessor des mobilen Roboters, eines Antriebssystems des mobilen Roboters zum Bewegen oder Anhalten des mobilen Roboters durch mindestens ein Element ausgewählt aus der Gruppe bestehend aus: autonomem Bewegen oder Anhalten des mobilen Roboters und Bewegen oder Anhalten des mobilen Roboters durch über eine Kommunikationsschnittstelle entlang einer Desinfektionsroute in dem Bereich empfangene Steuersignale.

3. Verfahren nach Anspruch 2, wobei das Emittieren des UV-Lichts zum Desinfizieren mindestens eines Teils des Bereichs Folgendes beinhaltet:
Emittieren von UV-Licht, während sich der mobile Roboter entlang der Desinfektionsroute bewegt.

4. Verfahren nach Anspruch 1, das ferner Folgendes beinhaltet:
Zuweisen, an dem Prozessor des mobilen Roboters, von Positionen der ein oder mehreren Belichtungssensoren auf der Karte des Bereichs.

5. Verfahren nach Anspruch 1, wobei die Desinfektionsgrade der ein oder mehreren Belichtungssensoren während mindestens eines Zeitraums empfangen werden, der ausgewählt ist aus der Gruppe bestehend aus: vor der Emission von UV-Licht in dem Bereich, während der Emission von UV-Licht in dem Bereich und nach der Emission von UV-Licht in dem Bereich.

6. Verfahren nach Anspruch 1, das ferner Folgendes beinhaltet:
Zuweisen, an dem mobilen Roboter, einer tatsächlichen Belichtungsmenge der ein oder mehreren Belichtungssensoren mit dem UV-Licht auf der Basis von mindestens einem der empfangenen Bilder und der Desinfektionsgrade; und
Bestimmen, an dem mobilen Roboter oder dem Fernprozessor, eines zukünftigen Desinfektionsplans für den mobilen Roboter auf der Basis eines Vergleichs zwischen dem Belichtungsdiagramm und mindestens einem der empfangenen Bilder und den Desinfektionsgraden.

7. Verfahren nach Anspruch 1, das ferner Folgendes beinhaltet:
Bestimmen, an dem mobilen Roboter, ob die Emission von UV-Licht fortgesetzt werden soll, auf der Basis der Bilder der ein oder mehreren Belichtungssensoren und/oder der von den ein oder mehreren Belichtungssensoren gemessenen Desinfektionsgraden; und
Bestimmen, an dem mobilen Roboter oder dem Fernprozessor, eines zukünftigen Desinfektionsplans für den mobilen Roboter auf der Basis eines Vergleichs zwischen dem Belichtungsdiagramm und den Bildern der ein oder mehreren Belichtungssensoren und/oder den von den ein oder mehreren Belichtungssensoren gemessenen Desinfektionsgraden.

8. Verfahren nach Anspruch 1, das ferner Folgendes beinhaltet:
Bestimmen einer abgegebenen Dosis von UV-Licht auf der Basis von mindestens einem Element ausgewählt aus der Gruppe bestehend aus: den empfangenen Bildern der ein oder mehreren Belichtungssensoren und den empfangenen Desinfektionsgraden.

9. Verfahren nach Anspruch 1, das ferner Folgendes beinhaltet:
Vergleichen, an dem Prozessor des mobilen Roboters oder einem mit dem mobilen Roboter kommunikativ gekoppelten Serversystem, einer bestimmten abgegebenen Dosis mit dem Belichtungsdiagramm;
Speichern eines Ergebnisses des Vergleichs in einem mit mindestens einem Element ausgewählt aus der Gruppe bestehend aus dem mobilen Roboter und dem Serversystem kommunikativ gekoppelten Speicher; und
Bestimmen, an dem mobilen Roboter oder dem Fernprozessor, eines zukünftigen Desinfektionsplans für den mobilen Roboter auf der Basis des gespeicherten Vergleichs.

## Revendications

1. Procédé comprenant :
une émission, à l'aide d'une source de lumière d'un robot mobile, d'une lumière ultraviolette, UV, pour désinfecter au moins une partie d'une zone, comprenant l'au moins un élément parmi une surface et un objet, dans lequel la lumière UV est émise sur un ou plusieurs capteurs d'exposition disposés dans la zone ;
un tracé, à l'aide d'un processeur du robot mobile, d'une représentation de l'émission de la lumière UV sur une cartographie de la zone pour générer un tracé d'exposition, dans lequel la représentation est celle de la lumière UV émise sur au moins un élément parmi la surface, l'objet, et les un ou plusieurs capteurs d'exposition dans la zone, ladite représentation de l'émission de la lumière UV est basée sur un modèle d'émission de lumière utilisé par le processeur ;
une réception, au niveau du robot mobile, d'au moins un élément parmi le groupe constitué : d'images des un ou plusieurs capteurs d'exposition, et de niveaux de désinfection mesurés par les un ou plusieurs capteurs d'exposition, les un ou plusieurs capteurs d'exposition changent de couleur ou délivrent une valeur de dosage sur la base d'un dosage de lumière UV reçue ; et
une génération, à l'aide d'au moins un élément sélectionné parmi le groupe constitué :
(i) du robot mobile, et
(ii) d'un processeur distant
d'un rapport de validation **caractérisé en ce qu'**il comprend au moins le tracé d'exposition, dans lequel le tracé d'exposition est validé par comparaison du tracé d'exposition et d'au moins un élément sélectionné parmi le groupe constitué : des images des un ou plusieurs capteurs d'exposition qui sont capturées par un capteur d'images du robot mobile, et des niveaux de désinfection mesurés par les un ou plusieurs capteurs d'exposition qui sont capturés par le capteur d'images ou reçus par une interface de réseau du robot mobile par l'intermédiaire d'une liaison de communication avec les un ou plusieurs capteurs d'exposition sur la base d'une exposition à la lumière UV émise par une source de lumière d'un robot mobile.

2. Procédé selon la revendication 1, comprenant en outre,
une commande, au niveau du processeur du robot mobile, d'un système d'entraînement du robot mobile pour déplacer ou arrêter le robot mobile au moyen d'au moins un élément sélectionné parmi le groupe constitué de ce qui suit :
un déplacement ou un arrêt autonome du robot mobile, et un déplacement ou un arrêt du robot mobile par des signaux de commande reçus par l'intermédiaire d'une interface de communication avec un trajet de désinfection dans la zone.

3. Procédé selon la revendication 2, dans lequel l'émission de la lumière UV pour désinfecter au moins une partie de la zone comprend :
une émission de lumière UV à mesure que le robot mobile se déplace le long du trajet de désinfection.

4. Procédé selon la revendication 1, comprenant en outre :
une attribution, au niveau du processeur du robot mobile, de positions des un ou plusieurs capteurs d'exposition sur la cartographie de la zone.

5. Procédé selon la revendication 1, dans lequel les niveaux de désinfection des un ou plusieurs capteurs d'exposition sont reçus pendant au moins un temps sélectionné parmi le groupe constitué de ce qui suit : avant l'émission de lumière UV dans la zone, pendant l'émission de lumière UV dans la zone, et après l'émission de lumière UV dans la zone.

6. Procédé selon la revendication 1, comprenant en outre :
une attribution, au niveau du robot mobile, d'une quantité d'exposition réelle des un ou plusieurs capteurs d'exposition à la lumière UV sur la base d'au moins un élément parmi les images reçues et les niveaux de désinfection ; et
une détermination, au niveau du robot mobile ou du processeur distant, d'un futur plan de désinfection pour le robot mobile sur la base d'une comparaison entre le tracé d'exposition et au moins un élément parmi les images reçues et les niveaux de désinfection.

7. Procédé selon la revendication 1, comprenant en outre :
une détermination, au niveau du robot mobile, du fait de poursuivre une émission de lumière UV ou non sur la base de l'au moins un élément parmi les images des un ou plusieurs capteurs d'exposition et des niveaux de désinfection mesurés par les un ou plusieurs capteurs d'exposition ; et
une détermination, au niveau du robot mobile ou du processeur distant, d'un futur plan de désinfection pour le robot mobile sur la base d'une comparaison entre le tracé d'exposition et l'au moins un élément parmi les images des un ou plusieurs capteurs d'exposition et des niveaux de désinfection mesurés par les un ou plusieurs capteurs d'exposition.

8. Procédé selon la revendication 1, comprenant en outre :
une détermination d'une dose délivrée de lumière UV sur la base d'au moins un élément sélectionné parmi le groupe constitué : des images reçues des un ou plusieurs capteurs d'exposition, et des niveaux de désinfection reçus.

9. Procédé selon la revendication 1, comprenant en outre :
une comparaison, au niveau du processeur du robot mobile ou d'un système serveur couplé en communication avec le robot mobile, d'une dose délivrée déterminée avec le tracé d'exposition ;
un stockage d'un résultat de la comparaison dans une mémoire couplée en communication à au moins un élément sélectionné parmi le groupe constitué : du robot mobile, et du système serveur ; et
une détermination, au niveau du robot mobile ou du processeur distant, d'un futur plan de désinfection pour le robot mobile sur la base de la comparaison stockée.
